# EUROPÄISCHE PATENTANMELDUNG

(11) **EP 0 626 167 A2**
(43) Veröffentlichungstag der Anmeldung: **30.11.1994**
(21) Anmeldenummer: 94106389.3
(22) Anmeldetag: 25.04.1994
(51) Int. Cl.: A61K 7/40, A61K 7/48

(54) **Hautschutzschaum**

(30) Priorität: 28.05.1993 DE 4317780
(71) Anmelder: IG SPRÜHTECHNIK GMBH, D-79664 Wehr/Baden (DE)
(72) Erfinder: Guck, Franz, D-79618 Rheinfelden (DE)
(74) Vertreter: Flaccus, Rolf-Dieter, Dr.

(57) **Zusammenfassung**

Gegenstand der vorliegenden Erfindung ist ein Hautschutzschaum als Mittel gegen hautagressive Mittel.

## Beschreibung

Gegenstand der vorliegenden Erfindung ist ein Hautschutzschaum als Mittel gegen hautagressive Mittel.

Zahlreiche Menschen, insbesondere Industriearbeiter und -arbeiterinnen in der chemischen Industrie, in der Lackindustrie u.ä. kommen tagtäglich mit Chemikalien, Mineralölen, Lösungsmitteln, Laugen, Reinigungsmittel und agressiven Desinfektionsmitteln etc. in Berührung. Diese Substanzen tragen oftmals zu einer sehr starken Entfettung der Haut bei und haben nicht selten hautreizende Wirkung. Die betroffenen Personen leiden in der Folge oftmals unter rissigen und spröden Händen, ausgetrockneten Handflächen, verletzten Nagelbetten, allergischen Ekzemen, Kontaktekzemen und dyshidrotischen Ekzemen. Die Griffigkeit und der Tastsinn der Hände läßt nach, die Hände schmerzen bei der täglichen Arbeit.

Aufgabe der vorliegenden Erfindung war daher die Bereitstellung eines Hautschutzmittels, das die beschriebenen Auswirkungen von hautagressiven Mitteln unterdrückt bzw. verhindert.

Gelöst wurde die Aufgabe durch ein Hautschutzmittel in Form eines Schaumes, der auf die Haut aufgesprüht wird. Der Schutzschaum besteht aus einer wässrigen Emulsion, die zusammengesetzt ist aus:
- Fettsäureestern zur Hautspreizung mit guter Hautverträglichkeit und zur Herstellung eines wasserdampfdurchlässigen Films,
- mehrwertigen Alkoholen zur stabilen Dispersion der Wirkstoffe und Regulierung der Feuchtigkeit des Films,
- Emulgatoren zur Verbesserung der Schaumstabilität,
- Tensiden zur Verminderung der Oberflächenspannung und
- gegebenenfalls Neutralisationsmitteln für Harze und Emulgatoren.

Als Treibmittel können gasförmige Kohlenwasserstoffe verwendet werden, wie beispielsweise Propan, Butan oder Isobutan, sowie deren Mischungen.

Als Fettsäureester können beispielsweise Ester der Stearinsäure, Myristinsäure oder Palmitinsäure eingesetzt werden.

Mehrwertige Alkohole können beispielsweise sein: Glycerol, Propylenglykol oder Polyethylenglykol. Auch Mischungen dieser Alkohole können eingesetzt werden.

Verwendbare Emulgatoren können ausgewählt werden aus der Gruppe Palmitinsäure und/oder Sorbitansäureester.

Zur Verminderung der Oberflächenspannung einsetzbare Tenside sind Natriumlaurylethersulfate oder Amphotenside sowie deren Mischungen.

Als Neutralisationsmittel können beispielsweise Aminomethylpropanol oder Monoethanolamin, sowie deren Mischungen eingesetzt werden.

Als wirksam hat sich die nachfolgende Grundrezeptur herausgestellt:

| | |
|---|---|
| Fettsäureester | 5 - 10 % (Gew.-%) |
| mehrwertiger Alkohol | 1 - 3 % |
| Emulgator | 1 - 3 % |
| Tensid | 1 - 3 % |
| Neutralisationsmittel | 0 - 3 % |
| Treibmittel | 5 - 10 % |
| Wasser | ad 100 % |

Die hautschützende Wirkung kann durch die Zugabe von 1 bis 5 % Liposomen als Wirkstoffträger überraschenderweise noch einmal deutlich verbessert werden. Durch Verwendung der Liposomen werden die Wirkstoffe sehr gezielt an den Wirkort herangeführt. Die Substanzen des erfindungsgemäßen Hautschutzschaumes gelangen in deutlich erhöhter Weise in die unteren Hautschichten. Hierbei werden die Liposomen mit dem erfindungsgemäßen Mittel zunächst an das Keratin der Hornschicht der Haut gebunden. Die Hautoberfläche wird hierdurch lipophilisiert. Der entstehende Film vermindert den Wasserverlust der Haut, wodurch die Funktion der Haut deutlich verbessert wird. Ein Teil der eingesetzten Liposomen wird durch die erkennbare Affinität zum Keratin abgebaut. Wird ein so hoher Anteil an Liposomen zugegeben, daß sie nicht vollständig durch diesen ersten Schritt aufgezehrt werden, so durchdringen die mit den Wirkstoffen beladenen Liposomen auch die Hornschicht der Haut und gelangen so zu den Talgdrüsen. Hierdurch wird wiederum der Stoffwechsel der Zellen aktiviert, so daß der liposomenhaltige erfindungsgemäße Hautschutzschaum zu einem sehr hohen Pflegewert führt. Der Hauttrockenheit wird wirkungsvoll begegnet. Der durch Liposomen ergänzte Hautschutzschaum führt zu guten Wirkstoffkonzentrationen in der gesamten Hornschicht über einen deutlich längeren Zeitraum als ohne Liposomen. Die einsetzbaren Liposomen können beispielsweise aus Phospholipiden hergestellt werden.

Besonders wirksam ist eine Mischung aus:

| | |
|---|---|
| Stearat | 6,0 % (Gew.-%) |
| Glycerol | 1,0 % |
| Sorbitol | 0,5 % |
| Propylenglykol | 2,0 % |
| Liposomen | 5,0 % |
| Propan/Butan | 9,0 % |
| Wasser | ad 100 % |

Verwendet wird der Hautschutzschaum in folgender Weise: Die den erfindungsgemäßen Hautschutzschaum enthaltende Dose ist vor Gebrauch gut zu schütteln. Die zu schützende Haut sollte, soweit möglich, trocken sein. Eine etwa nußgroße Menge des Schaums wird gleichmäßig in die Haut eingerieben. Nach etwa 2 Minuten Einwirkzeit bildet sich ein sehr wirkungsvoller, sicherer Hautschutz, der einen mehrstündigen Schutz gegen Berührungsallergien und andere hautagressiven Stoffe bietet. Chemikalien und dessen Rückstände können sich durch den erfindungsgemäßen Kautschutz nicht mehr in der Haut festsetzen. Überraschenderweise lassen sich selbst Lackrückstände leichter von den verunreinigten Hautpartien lösen. Selbst Laborarbeiten, die bisher das Tragen von Handschuhen zum Schutz gegen hautagressive Stoffe erforderlich machten, ließen sich bei Verwendung des erfindungsgemäßen Hautschutzschaumes ohne diese ausführen. Die Feinfühligkeit der Hände bleibt erhalten, ohne daß auf den Laborgeräten Rückstände des Mittels festzustellen waren. Überraschenderweise ließen sich selbst Arbeiten mit chlorierten Lösungsmitteln, wie beispielsweise mit Methylenchlorid/Dichlormethan weitestgehend ohne Handschuhe durchführen. Die Entfettung der Haut, sichtbar an den weißen Hautpartien, kann mit dem erfindungsgemäßen Mittel weitgehend vermieden werden. Entfernen läßt sich der Hautschutzschaum durch mehrmaliges intensives Waschen
Das erfindungsgemäße Mittel eignet sich insbesondere:
- als Schutz gegen wässrige Systeme aller Art, wie beispielsweise Säuren und Laugen, alkalische Entfetter- und Abbeizmittel, Zement und Kalk, Düngemittel, Wasch- und Spüllaugen, Desinfektionsmittel, Tinte, Tuschen usw.,
- als Schutz gegen wasserfreie Lösungsmittel, wie beispielsweise Aromaten, Aliphaten und halogenierte Kohlenwasserstoffe,
- als Schutz gegen Fette, Öle, Lackfarben, flüssige Kunststoffe und Bitumen,
- als Schutz gegen mechanische Einwirkungen von Glas- oder Mineralwolle,
- als Schutz gegen Berührungsallergien aller Art, sowie
- als Schutz gegen die Einwirkung von Kosmetikprodukten.

## Patentansprüche

1. Hautschutzschaum, bestehend aus einer wässrigen Emulsion aus:
- Fettsäureestern zur Hautspreizung mit guter Hautverträglichkeit und zur Herstellung eines wasserdampfdurchlässigen Films,
- mehrwertigen Alkoholen zur stabilen Dispersion der Wirkstoffe und Regulierung der Feuchtigkeit des Films,
- Emulgatoren zur Verbesserung der Schaumstabilität,
- Tensiden zur Verminderung der Oberflächenspannung und
- gegebenenfalls Neutralisationsmitteln für Harze und Emulgatoren.

2. Hautschutzschaum gemäß Anspruch 1, dadurch gekennzeichnet, daß zusätzlich Liposomen in Mengen von 1 bis 5 % enthalten sind.

3. Hautschutzschaum gemäß einem der Ansprüche 1 oder 2, dadurch gekennzeichnet, daß als Fettsäureester Myristinsäure oder Palmitinsäure in Mengen von 5 bis 10 %, als mehrwertige Alkohole Glycerol, Propylenglykol oder Polyethylenglykol in Mengen von 1 bis 3 %, als Emulgatoren Palmitinsäureester oder Sorbitansäureester in Mengen von 1 bis 3 %, als Tenside Natriumlaurylethersulfate oder Amphotenside in Mengen von 1 bis 3 % und als Neutralisationsmittel Aminomethylpropanol oder Monoethanolamin in Mengen von 0 bis 3 % eingesetzt werden.

4. Hautschutzschaum gemäß einem der vorhergehenden Ansprüche, dadurch gekennzeichnet, daß er aus:
| | |
|---|---|
| Stearat | 6,0 % (Gew.-%) |
| Glycerol | 1,0 % |
| Sorbitol | 0,5 % |
| Propylenglykol | 2,0 % |
| Liposomen | 5,0 % (Gew.-%) |
| Propan/Butan | 9,0 % |
| Wasser | ad 100 % |
besteht.

5. Verwendung des Hautschutzschaumes gemäß einem der vorhergehenden Ansprüche:
- als Schutz gegen wässrige Systeme aller Art, wie beispielsweise Säuren und Laugen, alkalische Entfetter- und Abbeizmittel, Zement und Kalk, Düngemittel, Wasch- und Spüllaugen, Desinfektionsmittel, Tinte, Tuschen usw.,
- als Schutz gegen wasserfreie Lösungsmittel, wie beispielsweise Aromaten, Aliphaten und halogenierte Kohlenwasserstoffe,
- als Schutz gegen Fette, Öle, Lackfarben, flüssige Kunststoffe und Bitumen,
- als Schutz gegen mechanische Einwirkungen voll Glas- oder Mineralwolle,
- als Schutz gegen Berührungsallergien aller Art oder
- als Schutz gegen die Einwirkung von Kosmetikprodukten.
